# EUROPEAN PATENT APPLICATION

(11) **EP 2 359 909 A2**
(43) Date of publication of application: **24.08.2011**
(21) Application number: 10252099.6
(22) Date of filing: 13.12.2010
(51) Int. Cl.: A61Q 5/06, A61Q 5/10, A61K 8/19, A61K 8/64

(54) **Method for selectively treating hair**

(30) Priority: 14.12.2009 US 637325
(71) Applicant: Johnson & Johnson Consumer Companies, Inc., Skillman, NJ 08558 (US)
(72) Inventor: Daly, Susan, Basking Ridge, NJ 07920 (US)
(74) Representative: Carpmaels & Ransford

(57) **Abstract**

This invention relates to methods and kits for selectively depositing a treatment agent on hair without depositing the treatment agent on the skin. The method involves sequentially treating the hair with a cationic compound selected from cationic peptides, cationic proteins, cationic polymers, cationic dyes, cationic pigments, or mixtures of these in a composition with a pH from about 3 to about 6, followed by treatment with an anionic treatment compound selected from anionic particles, polymers, proteins, peptides, dyes, or mixtures of these in a composition with a pH of from about 5.5 to about 12. By this method, the treatment agent becomes deposited on the hair; however, any skin that is also contacted by the treatment compositions remains substantially untreated.

## Description

### FIELD OF THE INVENTION

This invention relates to methods for selectively depositing a treatment agent on hair without depositing the treatment agent on the skin. The method involves sequentially treating the hair with a cationic peptide, protein, polymer, dye or pigment, or mixtures thereof in a composition with a pH from about 3 to about 6 followed by treatment with an anionic treatment particle in a composition with a pH from about 5.5 to about 12. By this method, the treatment agent becomes deposited on the hair; however, any skin that is also contacted by the treatment compositions remains untreated.

### BACKGROUND OF THE INVENTION

For many years, individuals have used cosmetic care and treatment compositions on their hair in an effort to protect, enhance and/or change certain characteristics of their hair, such as color, manageability, texture, and protection from environmental effects. Most of these care and treatment compositions function by either coating the hair with a treatment composition, as with conditioning agents, or by chemically altering the hair structure or surface, such as occurs with the use of most hair coloring agents. However, most hair care and treatment compositions are not selective to hair; that is, they also have some effect on other keratin-containing surfaces, such as skin, with which they come into contact when they are being applied to the hair. In some cases, this effect on skin is undesirable and care must be taken to limit contact of the treatment composition to the hair only and avoid contact with the skin.

An example is existing hair coloring technology, which generally relies on the use of permanent or semi-permanent dyes to accomplish this color change. Such dyes generally operate under oxidative and direct dyeing processes and cause substantial damage to the hair of individuals utilizing such dyes. Furthermore, such dyes also stain the skin and may cause skin irritation. After this happens, some users must simply wait for the stained and/or irritated skin to slough off or heal.

To mitigate this problem of skin staining, some hair coloring products are sold in conjunction with other products that are applied to the skin prevent dyeing of the skin or that chemically remove the dye after it has already deposited onto the skin. However, these prevention and removal products may further irritate the skin; they also add another step to the already laborious process of hair coloring. In spite of these disadvantages, this process has come to be the most acceptable means of achieving desired shades and colors in hair.

Pigment, defined as a "fine insoluble white, black, or colored material" [Julius Grant, editor, Hackh's Chemical Dictionary, McGraw-Hill Book Company, New York, 1969], has also been utilized to change the color of substrates. However, pigments are rarely used in hair coloring due to the complexities of applying and retaining the pigment on hair. The geometry of pigment particles, their size, index of refraction, and surface properties are among the characteristics of pigments that make them difficult to use in hair coloring processes.Because most pigments or colored particles are anionic in charge, they do not deposit readily onto anionically charged surfaces, such as keratin-containing substrates.

While pigments are used in mascara, they are present in these compositions in very high loadings, on the order of 10 to 30 weight percent. Furthermore, pigments must be held in place using film-forming polymers or other styling polymers. These compositions must coat the hair and adhere the pigments to the hair surface. These mechanical bonds are fairly loose and thus such pigment compositions are quite easy to remove. Coating longer hairs with polymers may also cause the hairs' texture and appearance to become unnatural and result in difficulty in managing the hair.

Thus, heretofore, there has not been means for selectively treating hair by sustainably attaching treatment agents to the hairs while simultaneously preventing attachment of the treatment agents to the skin.

### SUMMARY OF THE INVENTION

Surprisingly, it has been found that by exposing the hair and skin to a first composition containing a cationic compound and having a pH of between 3 and 6 and a preferably low ionic strength results in selective deposition of the compound onto hair and not skin. Furthermore, it has been found that by then exposing the hair and skin to a second composition containing an anionic treatment agent and having a pH of between 5.5 and 12 and a preferable ionic strength between about 5 and about 125 mM and more preferably between about 10 mM and about 25mM, the anionic treatment agent is also deposited only on the exposed hair without depositing on the exposed skin.

This invention relates to methods and kits for selectively depositing a treatment agent on hair without depositing the treatment agent on the skin. The method involves sequentially treating the hair with a cationic compound selected from cationic peptides, cationic proteins, cationic polymers, cationic dyes, cationic pigments, or mixtures of these in a composition with a pH from about 3 to about 6, followed by treatment with an anionic treatment compound selected from anionic particles, polymers, proteins, peptides, dyes, or mixtures of these in a composition with a pH of from about 5.5 to about 12. By this method, the treatment agent becomes deposited on the hair; however, any skin that is also contacted by the treatment compositions remains substantially untreated.

Accordingly, a method is provided for selectively depositing materials onto hair of a mammal and not onto skin of a mammal comprising sequentially:
a) providing a first cosmetic composition containing at least one cationic compound selected from the group consisting of cationic peptides, cationic proteins,and cationic polymers, cationic dyes or cationic pigments may also be utilized if a second layer is not intended to be applied, where the first cosmetic composition has a pH of from about 3 to about 6;
b) applying the first cosmetic composition to a hair for a time period sufficient for at least one said cationic compound to be deposited on the hair and form a layer;
c) optionally rinsing the first cosmetic composition from the hair with water;
d) optionally, providing a second cosmetic composition containing at least one anionic treatment agent, where the second cosmetic composition has a pH of from about 5.5 to about 12;
e) optionally, applying the second cosmetic composition to the hair for a time period sufficient for the anionic treatment agent to be deposited on the layer; and
f) optionally, rinsing the second cosmetic composition with water.

The sequential application of the first and second cosmetic compositions of the invention may be repeated one or more times to selectively deposit additional layers onto the hair. Additional layers may provide enhanced treatment and improved resistance to wash-out by surfactant treatment or cleansing.

The invention also provides a cosmetic kit for selectively coloring a hair comprising:
a) a first container containing a first cosmetic composition having a pH of from about 3 to about 6, comprising at least one cationic peptide;
   wherein said first composition is applied to the hair for a time period sufficient for at least one said cationic peptide to be deposited on the hair and form a layer, and the remaining first cosmetic composition is rinsed off with water;
b) a second container containing a second cosmetic composition having a pH of from about 5.5 to about 12, comprising at least one anionic pigment,
   wherein said second cosmetic composition is applied to the hair for a time period sufficient for the at least one anionic pigment to be deposited on the layer formed from the first composition, and the remaining second composition is rinsed off with water.

Other features and advantages of this invention will be apparent from the detailed description of the invention and from the claims.

### BRIEF DESCRIPTION OF THE DRAWINGS

Figure 1 is a chart of streaming potential, illustrating the results obtained in Example 1.
Figure 2 is a photograph of skin samples treated as described in Example 1.
Figure 3 is a chart of streaming potential, illustrating the results of deposition of polylysine on hair at pH 4.5, and illustrating the results of deposition of polylysine on hair at pH 7.5, as described in Example 2.
Figure 4 is a bar graph of the streaming potential shift (Δ streaming potential) as described in Example 3.
Figure 5 is a photograph showing hair samples and skin samples treated as described in Example 4.

### DETAILED DESCRIPTION OF THE INVENTION

The method and kits of this invention provide selective deposition of cationic treatment agents to hair, without deposition of the treatment agents onto exposed skin, by deposition of a cationically charged agent within a defined pH range. Unexpectedly, it was found that controlling the pH of the compositions from which the cationic compounds were deposited resulted in deposition only onto the hair, leaving the skin free of deposition. Thus, the methods of this invention may be used for selectively depositing cationic treatment agents, (including, but not limited to peptides, polymers, proteins, hair dyes, pigments and the like), onto hair and not skin.

The methods and kits of this invention provide selective deposition of anionic treatment agents to hair, without deposition of the treatment agents to any exposed skin, by the sequential deposition of a cationically charged compound layer onto the hair followed by the deposition of an anionically charged treatment agent onto the hair. Unexpectedly, it was found that controlling the pH of the compositions from which the cationic compounds (peptides, proteins, or polymers) and the anionic treatment agents were deposited resulted in deposition only onto the hair, leaving the skin free of deposition. Thus, the method of the invention may be used for selectively depositing treatment agents, including colored pigments or particles onto hair.

The initial portion of the method of the invention involves providing a first cosmetic composition containing a net positively charged (i.e., cationic) compound, which may be a cationic peptide, a cationic protein, or a cationic polymer, and applying this first composition to a hair to form a first layer on the hair. The first composition has a pH of between about 3 and about 6. This first layer does not form on any skin that is also exposed to the first composition. Without wishing to be bound by theory, it is believed that the reason for the selective deposition of the cationic compound lies in the differences in the surface properties, including surface charge, of the hair and the skin.

The next portion of the invention involves providing a second cosmetic composition containing an anionically charged treatment agent, and sequentially applying this second composition to the hair. The second cosmetic composition has a pH between about 5.5 and about 12 and a salt concentration of between about 5 and about 125 mM. The application of the second composition results in the deposition of a treatment agent layer onto the earlier deposited first layer. However, no treatment agent layer is deposited on surfaces, such as skin, that did not receive deposition of the first layer.

It is believed that one skilled in the art can, based upon the description herein, utilize the compositions and methods of this invention to their fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. Unless otherwise indicated, a percentage refers to a percentage by weight (i.e., %(W/W)).

### Definitions

"Hair", as used herein, relates to any keratin-containing fiber or fibrous material, including any human scalp or body hair, animal hair, wool, and fur.

"Skin", as used herein, relates to any of the outer, epidermal covering of the body, including, without limitation, the scalp, the lips, and the mucous membranes.

"Cationic", as used herein, is used to describe a compound or material with a positive charge. Such compounds generally move toward the negative electrode in electrolysis.

"Anionic", as used herein, is used to describe a compound or material with a negative charge. Such compounds generally move toward the positive electrode in electrolysis.

"Peptide", as used herein, is a molecule containing two or more amino acids joined by a peptide bond or modified peptide bonds.

The term "amino acid" refers to the basic chemical structural unit of a protein or polypeptide. The following abbreviations are used herein to identify specific amino acids:

**Table 1**

| **Amino Acid** | **Three-Letter Abbreviation** | **One-Letter Abbreviation** |
|---|---|---|
| Alanine | Ala | A |
| Arginine | Arg | R |
| Asparagine | Asn | N |
| Aspartic acid | Asp | D |
| Cysteine | Cys | C |
| Glutamine | Gln | Q |
| Glutamic acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

"Protein", as used herein, relates to a long chain of amino acids joined together by peptide bonds and having a molecular weight of greater than 10,000 Da.

"Polymer", as used herein, relates to a large organic molecule formed by combining many smaller molecules (monomers) in a regular pattern.

"Treatment agent", as used herein, relates to any compound that may be used to produce a desirable cosmetic effect on hair.

"Isoelectric point" or "IEP", as used herein, relates to the pH of a solution in which a charged molecule does not migrate in an electric field.

"Particle", as used herein, refers to a small, discrete portion of material that has mass and dimension.

"Microparticle", as used herein, refers to a particle having a diameter ranging from about 1 to about 1000 micrometers.

"Nanoparticle", as used herein, refers to a particle having a diameter ranging from about 1 to about 1000 nanometers.

"Pigment", as used herein, refers to a fine, insoluble white, black or colored material. For the purposes of this invention, pigments also include pigment particles, pigment microparticles, and pigment nanoparticles.

"Molar ratio", as used herein, relates to the ratio of the molecular weight of a specific portion of a molecule to the molecular weight of the whole molecule.

"Diameter", as used herein, refers to the largest side-to-side linear dimension of a particle, microparticle, or nanoparticle.

"Zeta potential", as used herein, relates to an electrokinetic measurement in a colloidal system. Zeta potential is the difference in potential between the dispersion medium and the immovable layer attached to the surface of the dispersed phase. Zeta potential may be measured using a Malvern zetasizer.

### Cationic compounds

The cationic compounds useful in the compositions and methods of this invention include cationic proteins, cationic peptides, cationic polymers, cationic dyes, cationic pigments and mixtures of these.

Cationic proteins include naturally-occurring cationic proteins and synthetic cationic proteins. Examples of naturally-occurring cationic proteins include lysozyme; avidin; methylated collagen; Cytochrome C; Platelet Factor 4; Protamine sulfate; Telomerase; cationic proteases, including trypsin, chymotrypsin, papain, caspase; RNA or DNA binding proteins, including histones, Ribonuclease A, and Deoxyribonuclease (DNase); and antimicrobial proteins, including magainin, defensins, and cathelicdin. Examples of cationic synthetic proteins include polylysine, polyarginine, polyhistidine, copolymers of these, or proteins containing a molar ratio of 50% or more of lysine, arginine, or histidine amino acids.

Synthetic cationic peptides include, for example, polylysine, polyarginine, polyhistidine, copolymers of these, or peptides containing a molar ratio of 50% or more of lysine, arginine, or histidine amino acids.

Cationic polymers include naturally-occurring cationic polymers and synthetic cationic polymers. Examples of naturally-occurring cationic polymers include, without mi limitation, chitosan, polyquaternium-4, polyquaternium-10, polyquaternium-24, and modifications of these.

Examples of synthetic cationic polymers include, without limitation, synthetic cationic polymers with one or more primary amines, synthetic cationic polymers with one or more secondary amines, synthetic cationic polymers with one or more tertiary amines, synthetic cationic polymers with one or more quaternary amines, and mixtures of these. Specific examples of synthetic cationic polymers include, without limitation, homopolymers or copolymers derived from acrylic or methacrylic esters or amides, such as poly methacrylamidopropyltrimethylammonium chloride, polyquaternium-1, polyquaternium-2, polyquaternium-5, polyquaternium-6, polyquatenium-7, polyquaternium-8, polyquaternium-11, polyquaternium-16, polyquaternium-17, polyquaternium-18, polyquaternium-22, polyquaternium-27, polyquaternium-28, polyquaternium 31, polyquaternium-39, polyquaternium-43, polyquaternium-44, polyquaternium-46, polyquaaternium-47, polyquaternium-53, polyquaternium-55, polyvinylpyrrolidone (PVP)/dimethylaminoethyl methacrylate copolymer, vinyl pyrrolidone (VP)/dimethylaminoethyl methacrylate copolymer, VP/dimethylaminopropylamine (DMAPA) acrylate copolymer, VP/vinyl caprolactam/ DMAPA acrylate copolymer, vinylcaprolactam/PVP/dimethylaminoethylmethacrylate copolymer, mixtures of these, and the like.

Non-limiting examples of cationic dyes contained in the composition of this invention may include basic dyes, cationic anthroquinonic dyes, azo dyes or qunone-imine dyes having a quaternary ammonium group, or dyes belonging to the Arianor semi-permanent hair dye family available from LCW/Sensient (South Plainfield, NJ). rin

Non-limiting examples of cationic pigments contained in the composition of this invention may include metal compounds or semimetallic compounds in ionic, nonionic or oxidized form. The pigments can be in this form either individually, in a mixture, or as individual mixed oxides or mixtures thereof, including mixtures of mixed oxides and pure oxides. Examples are the titanium oxides (for example TiO₂), zinc oxides (for example ZnO), aluminum oxides (for example Al₂O₃), iron oxides (for example Fe₂O₃), manganese oxides (for example MnO), silicon oxides (for example SiO₂), silicates, cerium oxide, zirconium oxides (for example ZrO₂), barium sulfate (BaSO₄) or mixtures thereof. Suitable pigments are commercially available. An example is Red Iron Oxide LC381 supplied by Sensient (South Plainfield, NJ).

The cationic compounds of this invention preferably have an Isoelectric Point of from about 6 to about 12, preferably from about 7.5 to about 12.

The cationic compounds used in this invention have a concentration range of from about 0.000001% to about 10% by weight, more preferably from about 0.001% to about 5% by weight, and even more preferably from about 0.01% to about 2% by weight.

### Anionic treatment agents

The anionic treatment agents contained in the second composition of this invention can be any anionically charged compounds useful for imparting a cosmetically desirable effect on hair. Nonlimiting examples include anionic proteins, anionic peptides, anionic polymers, anionic particles, anionic dyes and mixtures of these.

Anionic proteins include naturally-occurring anionic proteins and synthetic anionic proteins. Examples of naturally-occurring anionic proteins include, without limitation, wheat acidic esterase; alkaline phosphatase; beta-galactosidase; lactase; lipase; amylases; Epidermal Growth Factor; glycosidases; glucose oxidase; nitrate reductase; catalase; lactoglobulin; carboanhydrase; casein proteins from milk; trypsin inhibitor; albumin; anionic proteases, such as cathepsin; proteins from egg white, including ovalbumin, gamma-globulin, and ovomucin.

Synthetic anionic proteins include, for example, polyglutamic acid, polyaspartic acid, copolymers of these materials, and proteins containing a molar ratio of 50% or more of glutamic acid or aspartic acid amino acids.

Examples of anionic peptides include, without limitation, polyglutamic acid, polyaspartic acid, copolymers of these materials, and peptides containing a molar ratio of 50% or more of glutamic acid or aspartic acid amino acids.

Anionic polymers include naturally-occurring anionic polymers and synthetic anionic polymers. Examples of naturally-occurring anionic polymers include, without limitation, alginic acid, propylene glycol alginate, carrageenan gum, cellulose gum, gum acacia, karaya gum, xanthan gum, tragacanth gum, hyaluronic acid, shellac, and mixtures of these.

Nonlimiting examples of synthetic anionic polymers include sodium laureth sulfate (SLES), sodium polystyrene sulfonate, sodium polymethacrylate, sodium polynapthalenesulphonate, acrylates/C10-30 alkyl acrylate crosspolymer, acrylates/beheneth-25 methacrylate copolymer, acrylates/steareth-20 methacrylate copolymer, acrylates/ Lya vinyl acetate(VA)crosspolymer, acrylic acid/acrylonitrogens copolymer, carbomer polyvinylmethyl ether(PVM)/methylacrylate(MA)decadiene crosspolymer, acrylates copolymer, octylacrylamide/acrylates/ butylaminoethylmethacrylate copolymer, PVM/MA copolymer, VA/crotonates/vinyl neodecanoate copolymer, glyceryl polymethacrylate, and mixtures of these.

Non-limiting examples of anionic dyes contained in the composition of this invention may include FD&C Red 40, FD&C Yellow 6, FD&C Red #4, Tartrazine, Orange B and mixtures thereof.

The anionic particles contained in the second composition of this invention can be anionic pigments, other anionic particles, such as microparticles or nanoparticles, or combinations of these.

The anionic pigments contained in the second composition of this invention can be anionically charged colored pigment particles, microparticles or nanoparticles, or combinations of these. Preferred for use in this invention are anionic colored pigments.

Pigments, particularly metal compounds or semimetallic compounds, may be used in the compositions and methods of this invention in ionic, nonionic or oxidized form. The pigments can be in this form either individually or in admixture or as individual mixed oxides or mixtures thereof, including mixtures of mixed oxides and pure oxides. Examples are the titanium oxides (for example TiO₂), zinc oxides (for example ZnO), aluminum oxides (for example Al₂O₃), iron oxides (for example Fe₂O₃), manganese oxides (for example MnO), silicon oxides (for example SiO₂), silicates, cerium oxide, zirconium oxides (for example ZrO₂), barium sulfate (BaSO₄) or mixtures thereof. Suitable pigments are commercially available. An example is Hombitec® L5 (INCI name: titanium dioxides) supplied by Merck.

Other examples of pigments include the following: D&C Red No. 36, D&C Red No. 30, D&C Orange No. 17, Green 3 Lake, Ext. Yellow 7 Lake, Orange 4 Lake, Red 28 Lake, the calcium lakes of D&C Red Nos. 7, 11, 31 and 34, the barium lake of D&C Red No. 12, the strontium lake D&C Red No. 13, the aluminum lakes of FD&C Yellow No. 5 and No. 6, the aluminum lakes of FD&C No. 40, the aluminum lakes of D&C Red Nos. 21, 22, 27, and 28, the aluminum lakes of FD&C Blue No. 1, the aluminum lakes of D&C Orange No. 5, the aluminum lakes of D&C Yellow No. 10; the zirconium lake of D&C Red No. 33, Cromophthal® Yellow, Sunfast® Magenta, Sunfast® Blue, iron oxides, calcium carbonate, aluminum hydroxide, calcium sulfate, kaolin, ferric ammonium ferrocyanide, magnesium carbonate, carmine, barium sulfate, mica, bismuth oxychloride, zinc stearate, manganese violet, chromium oxide, titanium dioxide, titanium dioxide nanoparticles, zinc oxide, barium oxide, ultramarine blue, bismuth citrate, hydroxyapatite, zirconium silicate, carbon black particles and the like.

The pigments or particles of this invention are anionically charged, either inherently or by virtue of an anionic coating. Suitable anionic coatings include, for example, silica, aluminosilicate, sodium C14-16 olefin sulfonate, disodium stearoyl glutamate, sodium stearoyl glutamate/sodium trideceth-6 carboxylate, and sodium polyacrylates/hydrogenated lecithin/aluminum hydroxide. Examples of anionically coated pigments suitable for use in the present invention are given in Table 1. nun

**Table 1**

| **Pigment Name** | **Chemical Name** | **Surface Coating** | **Source** |
|---|---|---|---|
| Sympholight RW | Iron Oxide | silica treated | Presperse, Inc. Somerset, NJ |
| Sympholight BW | Iron Oxide | silica treated | Presperse, Inc. Somerset, NJ |
| SP-4405 Surface Passivated Black Oxide | Iron Oxide | aluminosilicate | Color Techniques, Inc., South Plainfield NJ |
| SP-Surface Passivated titanium dioxide | Titanium Dioxide | aluminosilicate | Color Techniques, Inc., South Plainfield NJ |
| Sinert BP-10 | Iron Oxide | silica | Kobo Products, Inc. South Plainfield NJ |
| Sinert RP5-10 | Iron Oxide | silica | Kobo Products, Inc. South Plainfield NJ |
| BRO-C5 | Iron Oxide | silica | Kobo Products, Inc. South Plainfield NJ |
| Aquaspersabil RIO | Iron Oxide | sodium C₁₄₋₁₆ Olefin Sulfonate | Presperse, Inc. Somerset, NJ |
| Amino Acid (NAID) Treated Iron Oxide | Iron Oxide | Disodium Stearoyl Glutamate | U.S. Cosmetics Corporation, Dayville CT |
| BLACK BL-100 SPA | Iron Oxide | Sodium Stearoyl Glutamate (And) Sodium Trideceth-6 Carboxylate | Kobo Products, Inc. South Plainfield NJ |
| PALI Treated iron Oxide | Iron Oxide | Sodium Polyacrylates/ Hydrogenated Lecithin/ Aluminum Hydroxide | U.S. Cosmetics Corporation, Dayville CT |

The anionic pigments and particles preferred for use in this invention preferably have an Isoelectric Point of less than about 5, and preferably less than about 4.

The anionic pigments and particles used in this invention have a concentration range of from about 0.05% to about 10% by weight, more preferably from about 0.1% to about 5% by weight, and even more preferably from about 0.5% to about 2% by weight.

### Buffers

The pH of the first composition of this invention is between about 3 and about 6. Any cosmetically acceptable buffers may be used to adjust the pH of the composition to this desired range. Examples of such buffers include, without limitation, sodium citrate, 2-(N-morpholino)ethanesulfonic acid. Acetate, phosphate, lactate and other buffers known to a person skilled in the art having a buffer capacity between 3 and 6 are also useful in the compositions of this invention.

The pH of the second composition of this invention is between about 5.5 and about 12. Any cosmetically acceptable buffers may be used to adjust the pH of the composition to this desired range. Examples of such buffers include, without limitation, tris(hydroxymethyl)aminomethane (tris), MES buffers. The second composition of this invention also has a salt concentration of between about 5 and about 125 mM, preferably between about 10 and about 25 mM.

### Compositions

The compositions of this invention may be prepared in the form of formulations known to be useful for cosmetic hair products. For example, they can be in the form of shampoos, conditioners, lotions, rinses, dispersions, emulsions, gels, cream gels, creams, pastes, sticks, suspensions, sprays, mousse, aerosols, foams, or the like. To the compositions of the invention may be added other substances, auxiliary agents, for example those commonly used for cosmetic products in general. Such materials include, for example, thickeners (for example clays, starches, polyacrylic acid and the derivatives thereof), cellulose derivatives, lanolin derivatives, vitamins or provitamins, (for example biotin, vitamin C, tocopherols or D-panthenol), antigrease agents, inorganic or organic acids (for example lactic acid, citric acid, glycolic acid or phosphoric acid), preservatives (for example parahydroxybenzoate esters), nonaqueous solvents, antioxidants (for example tocopherols or the esters thereof), dyes and fragrances or perfumes, UV light-absorbing inorganic particles and others known to those of ordinary skill in the art.

### Other Cosmetic Components and Additives

In addition to the above-described ingredients, other common cosmetic components and additives may be incorporated in the compositions of this invention, as long as the basic properties of the compositions, including pH and salt concentration, are preserved. Such ingredients include, but are not limited to, humectants, emollients, moisturizers, inorganic salts, fragrances, hydrotropes, foam stabilizers, preservatives, water softening agents, and the like. Optional components may be present in weight percentages of less than about 2% each, and from about 5% to about 10% by weight of the composition in total.

### Cosmetically Acceptable Carriers:

The compositions of this invention preferably contain one or more cosmetically-acceptable carriers. Preferably, such carriers include water. Organic solvents may also be included in order to facilitate manufacturing of the composition or to provide esthetic properties, such as viscosity control. Suitable solvents include the lower alcohols like ethyl alcohol and isopropyl alcohol; glycol ethers, like 2-butoxyethanol, ethylene glycol monoethyl ether, propylene glycol and diethylene glycol monoethyl ether or monomethyl ether; and mixtures thereof. Nonaqueous solvents may be present in the compositions of the present invention in an amount of about 1% to about 50%, and in particular about 5% to about 25%, by weight of the total weight of the carrier in the composition.

The compositions of this invention should be stable to phase or ingredient separation at a temperature of about 25°C for an indefinite period of time, or at least for 5 weeks at a temperature of 45°C. Thus, the compositions of this invention have demonstrated sufficient stability to phase and ingredient separation at temperatures normally found in commercial product storage and shipping to remain unaffected for periods of at least one year.

The compositions of this invention may be utilized in any types of products that impart benefits to hair, including, but not limited to the following: hair conditioners, hair coloring, mascara, wool fabric coloring, and the like.

Treating the hair with the compositions of this invention is generally carried out by: (1) applying to dry or wet hair an effective amount of the first composition of the invention; (2) distributing the first composition of this invention more or less evenly throughout the hair such that it contacts all the hair or other substrate which is intended to be treated. This permits the cationic compound of the first compositions of this invention to be applied thoroughly and evenly throughout the hair or other substrate to form a layer on the hair. This step may be accomplished by rubbing the first composition throughout the hair manually or using a hair appliance such as a comb for up to about 20 minutes; and (3) rinsing said hair or other substrate with water so as to remove excess material that has not deposited onto the hair; (4) applying to dry or wet hair an effective amount of the second composition of the invention; (5) distributing the second composition of this invention more or less evenly throughout the hair such that it contacts all the hair or other substrate which is intended to be treated. This permits the treatment agent of the second composition of this invention to be applied thoroughly and evenly throughout the hair or other substrate. This step may be accomplished by rubbing the second composition throughout the hair manually or using a hair appliance such as a comb for up to about 20 minutes; and (6) rinsing said hair or other substrate with water so as to remove excess material that has not deposited onto the hair. Treating the hair with the compositions of the invention may also be carried out by applying leave-on types of compositions, such as hair spray, cream, or solution, directly to hair without rinsing the hair.

The compositions and methods of this invention are further defined in the following Examples. It should be understood that these Examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various uses and conditions.

### Streaming potential

Streaming potential is an electrokinetic measurement determined by passing an electrolytic solution through a permeable body, such as a capillary, a porous solid, or a plug of fiber such as hair. The streaming of the liquid through the permeable body produces an electrokinetic potential which may be measured. An electrometer may be used to measure the electrical potential across the sample caused by the flow of liquid. A detailed description of streaming potential can be found in US 5,452,233.

In the present invention, streaming potential is used to measure the surface charge on hair and on skin before and after treatments with certain compounds. Any change in streaming potential after treatment indicates a change in the surface charge of the hair or the skin, thus indicating deposition of a compound on the hair or skin. Therefore, the streaming potential measurement may be used to monitor the deposition and retention of the treatment compounds on the hair or the skin. The measurement may be illustrated as a graph where the x-axis represents time, measured in seconds in this invention, and the y-axis represents the streaming potential, measured in millivolts (mV) in this invention.

All human hair used in the examples of this invention was natural white hair in 250 mg tress samples. Such hair is available commercially, for example from International Hair Importers and Products (Bellerose, N.Y.), and is also available in different colors, such as brown, black, red, and blonde, and in various types, such as African-American, Caucasian, and Asian.

Skin samples used for testing in these examples consisted of tape samples each having a layer of human epidermal cells adhered thereto. The tape samples were prepared by adhering a clean sample of tape to the skin on a human forearm then removing and performing this step twice in order to obtain a second layer of skin. Upon removal, the outer layer of epidermal cells on the skin remained adhered to the tape adhesive. All testing solutions were applied to the tape samples to measure their effect on the adhered skin cells.

### Example 1

Streaming potential was used to follow polylysine adsorption to skin from a pH 7.5, 5 mM tris buffer solution. All streaming potential analyses in these examples were conducted using an Anton Paar SurPASS instrument.

Two solutions of 15 kDa polylysine (Sigma Catalog number P6516, Sigma-Aldrich, St. Louis, MO) were prepared as follows:
Solution 1 contained 0.0125 wt% of the polylysine in 5 mM tris solution in deionized water, with a pH of 7.5.
Solution 2 contained 0.0125 wt. % of the polylysine in 5 mM sodium citrate solution in deionized water with a pH of 4.5.

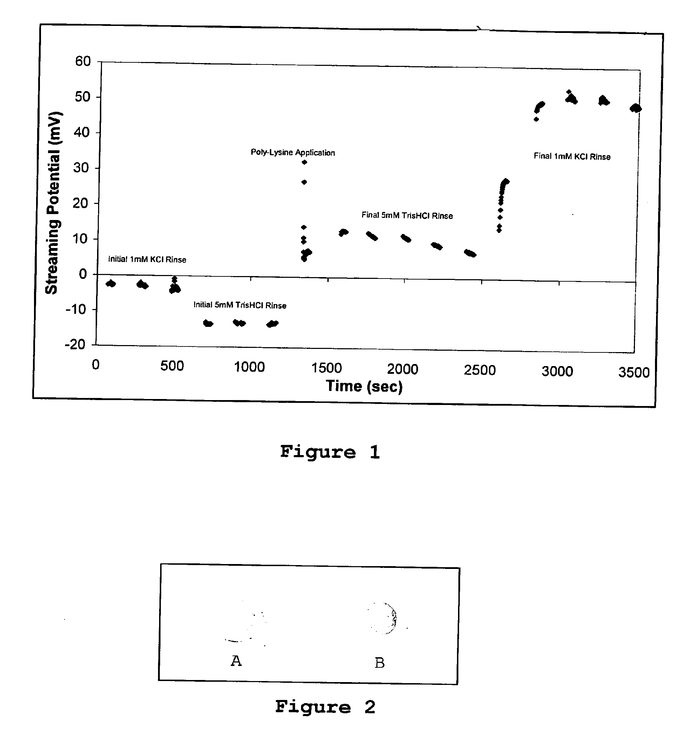

Streaming potential analysis of a skin sample was conducted using the 7.5 pH polylysine solution. Figure 1 shows the streaming potential for the skin treated with the pH 7.5 polylysine solution. Referring now to Figure 1, the first 3 data points, around -2 to -4 mV, correspond to streaming potential of the skin with an initial buffer rinse of 1mM KCl solution in deionized water. The next 3 data points correspond to initially rinsing the skin with a 5 mM tris/HCl buffer solution. The next data point corresponds to the application of Solution 1 above, the polylysine solution with pH 7.5. The next 5 data points correspond to the final rinsing with 5 mM tris buffer solution. The last 5 data points correspond to a final 1 mM KCl buffer rinse.

The streaming potential analysis of the skin shows differences in surface charge before and after the polylysine treatment, indicating that the polylysine adsorbs onto the skin when the skin is treated with the polylysine in a pH 7.5 solution.

After the streaming potential analysis was conducted, the skin sample was removed from the electrometer and treated with a solution of 0.25% (w/w) anionic colored pigment in 25 mM tris buffer solution with a pH of 7.5 for 10 minutes. The anionic colored pigment was a silica-coated red iron oxide with a 200 nm particle size. The pigment-treated sample was then rinsed with deionized water and photographed. As a comparison, a skin sample that had not been treated with the pH 7.5 polylysine solution was also treated with a solution of an anionic colored pigment in the same manner as the polylysine-treated skin sample. Both samples were then photographed. The photographs of both samples are shown in Figure 2. Referring now to Figure 2, it can be seen that the skin sample A has very little pigment deposited on it. Skin sample B is much darker, demonstrating that it has a significant amount of pigment deposited on it.

### Example 2

Streaming potential analysis was then conducted on a tape strip sample using the pH 4.5 polylysine solution, Solution 2 described in Example 1 above. The differences in streaming potential before and after treatment (Δ streaming potential) for each of the pH 7.5 and the pH 4.5 solutions are shown in Table 2 below.

**Table 2**

| Cationic Compound | Buffer | Solution pH | Δ Streaming Potential |
|---|---|---|---|
| 0.0125 wt% polylysine | 5 mM tris | 7.5 | 55 * |
| 0.0125 wt% polylysine | 5 mM citrate | 4.5 | 5 |

Referring to Table 2, it can be seen that, as the pH of the polylysine solution is decreased from 7.5 to 4.5, the Δ Streaming Potential of the skin decreases significantly, correlating to significantly decreased deposition of polylysine on the skin cells of the tape samples.

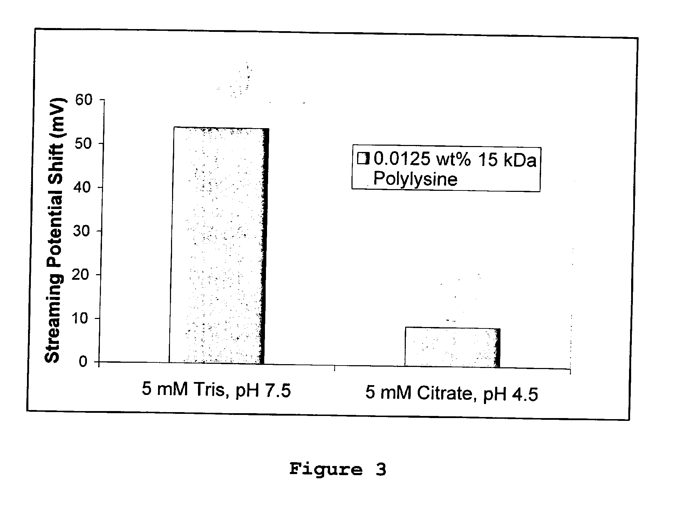

After the streaming potential analysis was conducted, the tape samples were removed from the electrometer and treated with a solution of 0.25% (w/w) anionic colored pigment in 25 mM tris buffer solution with a pH of 7.5 for 10 minutes. The samples were then rinsed with the 25 mM tris buffer and photographed. The anionic colored pigment was a silica-coated red iron oxide with a 200 nm particle size. The photographs are shown in Figure 3, along with a bar chart showing the data from Table 2 above, the Streaming Potential Shift (Δ Streaming Potential) of skin before and after treatment with each polylysine solution. Referring to Figure 3, it can be seen that the tape sample treated with the pH 7.5 solution was much darker than the sample treated with the pH 4.5 solution, confirming that more of the pigment deposited on the sample on which more of the polylysine had deposited.

### Example 3

Streaming potential analysis was then conducted on a hair samples using the pH 7.5 polylysine, Solution 1, and the 4.5 polylysine solution, Solution 2, as described in Example 1 above. The differences in streaming potential before and after treatment (Δ streaming potential) for each of the pH 7.5 and the pH 4.5 solutions are shown in Table 2 below.

**Table 3**

| Cationic Compound | Buffer | Solution pH | Δ Streaming Potential |
|---|---|---|---|
| 0.0125 wt% polylysine | 5 mM tris | 7.5 | 29 |
| 0.0125 wt% polylysine | 5 mM citrate | 4.5 | 26 |

Referring to Table 2, it can be seen that, as the pH of the polylysine solution is decreased from 7.5 to 4.5, the Δ Streaming Potential of the hair does not change significantly, correlating to a similar amount of deposition of polylysine on the two hair samples.

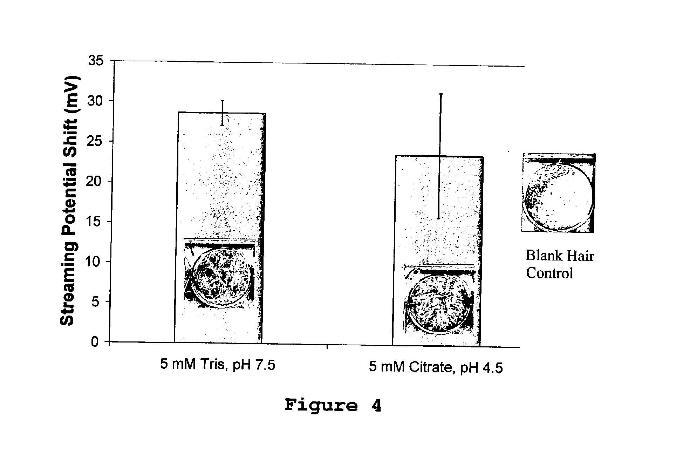

After the streaming potential analysis was conducted, the hair samples were removed from the electrometer and treated with a solution of 0.25% (w/w) anionic colored pigment in 25 mM tris buffer solution with a pH of 7.5. The samples were then rinsed with the 25 mM Tris buffer and photographed. The anionic colored pigment was a silica-coated red iron oxide with a 200 nm particle size. The photographs are shown in Figure 4, along with a bar chart showing the data from Table 3 above, the Streaming Potential Shift (Δ Streaming Potential) of skin before and after treatment with each polylysine solution. Referring to Figure 4, it can be seen that the hair samples were both colored by the pigment treatment, confirming that polylysine deposits onto hair at either pH 7.5 or pH 4.5.

### Example 4

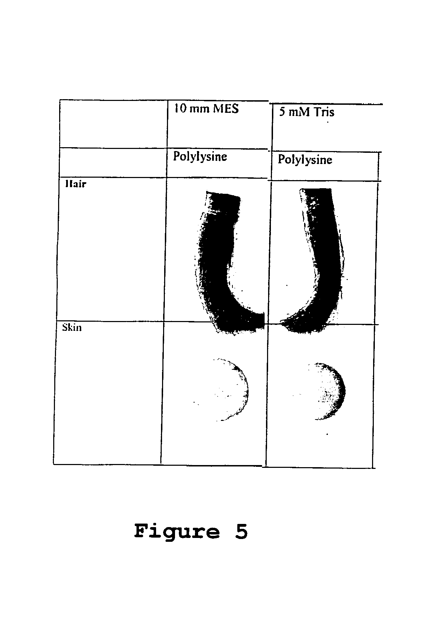

Hair tresses and tape stripped skin samples were also treated with 0.0125% (wt/wt) polylysine in pH 5.0, 10 mM MES [2-(N-morpholino)ethanesulfonic acid] buffer, Solution 3. The hair tresses were approximately 250 mg and comprised of 100% gray hair. Tresses were treated with 30 mL of peptide in either Solution 1 or Solution 3 for 10 minutes. Likewise, skin samples were treated with 5 mL of peptide in either Solution 1 or Solution 3 for 10 minutes. All samples were rinsed well with the corresponding peptide-free buffer and treated with a solution of 0.25% (w/w) anionic colored pigment in 25 mM tris buffer solution with a pH of 7.5. The samples were then rinsed with 25mM tris buffer solution and photographed. The anionic colored pigment was a silica-coated red iron oxide with a 200 nm particle size. The photographs are shown in Figure 5. Referring to Figure 5, it can be seen that the hair samples were both colored by the pigment treatment, confirming that polylysine deposits onto hair at either pH 7.5 or pH 4.5 whereas only the skin sample that was treated with peptide at pH 7.5 showed color deposition, confirming that we can selectively deposit a first layer of cationic material onto hair not skin and that we can use this selective first layer to selectively deposit a second layer of color or other active material onto hair not skin.

### Example 5

Hair tresses and tape stripped skin samples were treated with red iron oxide pigment in different buffering media. The red iron oxide pigment had an isoelectric point of pH 8. The hair tresses were approximately 250 mg in weight and contained 100% gray hair. Tresses were treated with 10 mL of 0.25% (w/w) red iron oxide in either (a) 10 mM MES [2-(N-morpholino)ethanesulfonic acid] pH 5 buffer, (b) 5 mM sodium citrate buffer pH 4 or (c) 5 mM Tris buffer, pH 7.5 for 30 minutes. Likewise, skin samples were treated with 2 mL of the 0.25% (w/w) red iron oxide in buffer solutions a-c for 30 minutes. All samples were rinsed well with the corresponding peptide-free buffer. Photographs were taken of the resulting hair and skin samples. The hair samples all colored by the pigment treatments, confirming that the cationic pigment deposits onto hair at all pH values examined (4, 5, 7.5), whereas only the skin sample that was treated with the pigment at pH 7.5 showed color deposition. Thus, by controlling the pH of the cationic treatment step, colored materials or other cationic molecules can be selectively deposited onto hair and not onto skin.

The specification and embodiments above are presented to aid in the complete and non-limiting understanding of the invention disclosed herein. Since many variations and embodiments of the invention can be made without departing from its spirit and scope, the invention resides in the claims hereinafter appended.

## Claims

1. A method for selectively depositing materials onto hair of a mammal and not onto skin of a mammal comprising sequentially:
a) providing a first cosmetic composition comprising at least one cationic material, wherein said first cosmetic composition has a pH of from about 3 to about 6;
b) applying said first cosmetic composition to a hair for a time period sufficient for at least one said cationic material to be deposited on the hair and form a layer,
whereby said cationic material is deposited on hair of a mammal and not on skin of a mammal.

2. A method according to claim 1 wherein said method further comprises the following steps:
c) rinsing said first cosmetic composition from the hair with water;
d) providing a second cosmetic composition comprising at least one anionic material, where said second cosmetic composition has a pH of from about 5.5 to about 12;
e) applying said second cosmetic composition to the hair for a time period sufficient for at least one said anionic material to be deposited on said layer; and
f) rinsing said second cosmetic composition with water.

3. A method according to claim 1 wherein said cationic material is selected from the group consisting of a cationic peptide, a cationic polymer, a cationic protein, a cationic dye, a cationic pigment and a combination thereof.

4. A method according to claim 3, wherein said cationic peptide comprising 50% or more of lysine, arginine, histidine amino acids, or the mixtures thereof.

5. A method according to claim 4, wherein said cationic material is a peptide selected from the group consisting of polylysine, polyarginine, polyhistidine and the mixtures thereof.

6. A method according to claim 1 or claim 3, claim 4 or claim 5, wherein said cationic material has an Isoelectric Point of from about 6 to about 12, or from about 7.5 to about 12.

7. A method according to claim 2, wherein said anionic material is a pigment having an isoelectric point of less than about 5.

8. A method according to claim 7, wherein said anionic pigment has an isoelectric point less than about 4.

9. A method according to claim 2 or claim 7 or claim 8, wherein said anionic material is a pigment selected from the group consisting of D&C Red No. 36, D&C Red No. 30, D&C Orange No. 17, Green 3 Lake, Ext. Yellow 7 Lake, Orange 4 Lake, Red 28 Lake, the calcium lakes of D&C Red Nos. 7, 11, 31 and 34, the barium lake of D&C Red No. 12, the strontium lake D&C Red No. 13, the aluminum lakes of FD&C Yellow No. 5 and No. 6, the aluminum lakes of FD&C No. 40, the aluminum lakes of D&C Red Nos. 21, 22, 27, and 28, the aluminum lakes of FD&C Blue No. 1, the aluminum lakes of D&C Orange No. 5, the aluminum lakes of D&C Yellow No. 10;
the zirconium lake of D&C Red No. 33, Cromophthal® Yellow, Sunfast® Magenta, Sunfast® Blue, iron oxides, calcium carbonate, aluminum hydroxide, calcium sulfate, kaolin, ferric ammonium ferrocyanide, magnesium carbonate, carmine, barium sulfate, mica, bismuth oxychloride, zinc stearate, manganese violet, chromium oxide, titanium dioxide, titanium dioxide nanoparticles, zinc oxide, barium oxide, ultramarine blue, bismuth citrate, hydroxyapatite, zirconium silicate, and carbon black particles.

10. A method according to claim 2, claim 7, claim 8, or claim 9, wherein said anionic material is an anionic microparticle.

11. A method according to claim 10, wherein said anionic microparticle has a diameter of from about 1 to about 1000 micrometers.

12. A method according to claim 2, claim 7, claim 8 or claim 9, wherein said anionic material is an anionic nanoparticle.

13. A method according to claim 12, wherein said anionic nanoparticle has a diameter of from about 1 to about 1000 nanometers.

14. A method according to any preceding claim, wherein said first cosmetic composition has a pH of from about 4 to about 5.5.

15. A method according to claim 2 or any one of claims 7 to 13, where said second cosmetic composition has a pH of from about 7.5 to about 12.

16. A method according to any preceding claim, wherein said hair is selected from the group consisting of hair, wool and fur.

17. A method according to any preceding claim, wherein said cationic material is a peptide having a concentration of from about 0.000001% to about 10% by weight, or from about 0.001% to about 5% by weight, or from about 0.01% to about 2% by weight.

18. A method according to claim 2, any one of claims 7 to 13 or claim 16, wherein said anionic pigment has a concentration of from about 0.05% to about 10% by weight, or from about 0.1% to about 5% by weight, or from about 0.5% to about 2% by weight.

19. A method according to any preceding claim wherein the ionic strength of the first cosmetic composition is less than about 150 mM, or less than about 25 mM, or the ionic strength of the second cosmetic composition is from about 1 to about 150 mM, or from about 10 to about 25 mM.

20. A cosmetic kit for selectively coloring a hair comprising:
a) a first container containing a first cosmetic composition having a pH of from about 3 to about 6, comprising at least one cationic peptide;
wherein said first composition is applied to the hair for a time period sufficient for at least one said cationic peptide to be deposited on the hair and form a layer, and the remaining first cosmetic composition is rinsed off with water;
b) a second container containing a second cosmetic composition having a pH of from about 5.5 to about 12, comprising at least one anionic pigment, wherein said second cosmetic composition is applied to the hair for a time period sufficient for the at least one anionic pigment to be deposited on the layer formed from the first composition, and the remaining second composition is rinsed off with water.

21. A colored hair obtainable by the method of any one of claims 1 to 19.

22. A colored hair according to claim 21, wherein said hair is selected from the group consisting of hair, wool and fur.
